# EUROPÄISCHE PATENTANMELDUNG

(11) **EP 0 726 259 A1**
(43) Veröffentlichungstag der Anmeldung: **14.08.1996**
(21) Anmeldenummer: 96101097.2
(22) Anmeldetag: 26.01.1996
(51) Int. Cl.: C07D 257/04

(54) **Verfahren zur Herstellung von substituierten N-Carbamoyl-tetrazolinonen**

(30) Priorität: 08.02.1995 DE 19504059
(71) Anmelder: BAYER AG, 51368 Leverkusen (DE)
(72) Erfinder: Stelzer, Uwe, Dr., D-51381 Leverkusen (DE); Gau, Wolfgang, Dr., D-42113 Wuppertal (DE); Reubke, Karl-Julius, Dr., D-51065 Köln (DE)

(57) **Zusammenfassung**

Die herbizid wirksamen substituierten N-Carbamoyltetrazolinone der Formel (I) 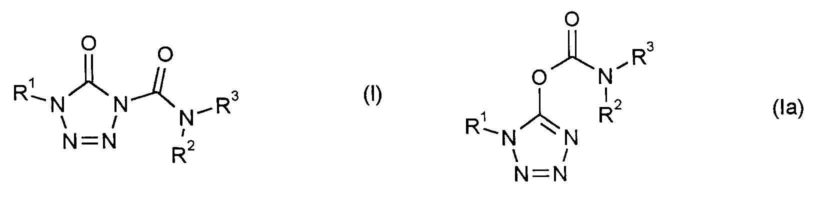 worin
- R¹: u.a. für einen (gegebenenfalls substituierten) Phenylrest,
- R²: u.a. für einen Alkylrest und
- R³: u.a. für einen Cycloalkylrest steht,
erhält man in sehr guten Ausbeuten und hoher Reinheit - d.h. frei von den isomeren O-Carbamoyloxy-tetrazolen (Ia) -, indem man Tetrazolinone der Formel (II) mit Carbamidsäurehalogeniden der Formel (III) (X = Halogen) 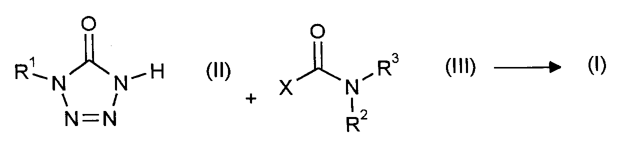 in Gegenwart eines Säureakzeptors und in Gegenwart eines Verdünnungsmittels bei Temperaturen zwischen 0°C und 200°C umsetzt und das hierbei als Nebenprodukt gebildete O-Carbamoylierungsprodukt der Formel (Ia) entweder durch Erhitzen zum gewünschten Produkt der Formel (I) isomerisiert oder durch Hydrolyse oder Alkoholyse in wasserlösliche und somit leicht abtrennbare Komponenten überführt (Variante (a)).

Drei mit diesem Verfahren (a) eng verwandte Varianten (b), (c) und (d) werden ebenfalls beschrieben:
Verfahrensvariante (b) verläuft analog zu (a), jedoch über die (neuen, isolierten) Metallsalze (IIa) der Tetrazolinone (II). Bei Variante (c) wird das Isomer (Ia), entweder in rein isolierter Form oder im Gemisch mit (I), thermisch isomerisiert, während bei Variante (d) das Isomer (Ia) aus (I)/(Ia)-Gemischen durch Hydrolyse oder Alkoholyse entfernt wird.

Die neuen Zwischenprodukte (Ia) und (IIa) als solche sind auch Gegenstand der Erfindung.

## Beschreibung

Die Erfindung betrifft ein neues Verfahren und neue Zwischenprodukte zur Herstellung von substituierten N-Carbamoyl-tetrazolinonen, welche als herbizid wirksame Verbindungen bekannt sind.

Es ist bekannt, daß man substituierte Carbamoyltetrazolinone erhält, wenn man entsprechende Tetrazolinone mit geeigneten Carbamidsäurederivaten umsetzt (vgl. EP-A 146279, EP-A 202929, EP-A 578090 und EP-A 612735). Bei dieser Herstellungsweise wird neben der erwünschten N-Carbamoylierung immer auch eine (unerwünschte) O-Carbamoylierung beobachtet (zur Acylierung von Tetrazolinonen vgl. auch Z. Chemie 13 (1973), 429-430). Man erhält also in vielen Fällen mehr oder weniger stark verunreinigte Produkte.

Es wurde nun gefunden, daß man substituierte N-Carbamoyl-tetrazolinone der allgemeinen Formel (I) 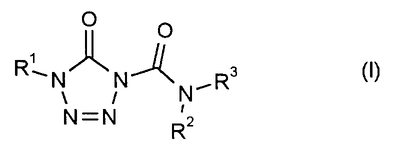 in welcher
- R¹: für jeweils gegebenenfalls substituiertes Alkyl, Alkenyl, Alkinyl, Cycloalkyl, Cycloalkylalkyl, Aryl, Arylalkyl, Heterocyclyl oder Heterocyclylalkyl steht,
- R²: für jeweils gegebenenfalls substituiertes Alkyl, Alkenyl, Alkinyl oder Alkoxy steht und
- R³: für jeweils gegebenenfalls substituiertes Alkyl, Alkenyl, Alkinyl, Cycloalkyl, Cycloalkylalkyl, Aryl oder Arylalkyl steht
oder zusammen mit R² für Alkandiyl steht,
in sehr guten Ausbeuten und in hoher Reinheit - d.h. frei von den isomeren Carbamoyloxytetrazolen (Ia) - erhält, wenn man
(a) Tetrazolinone der allgemeinen Formel (II) 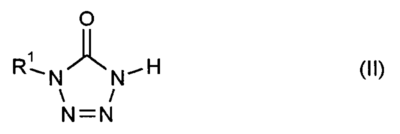 in welcher
   - R¹: die oben angegebene Bedeutung hat,
   mit Carbamidsäurehalogeniden der allgemeinen Formel (III) 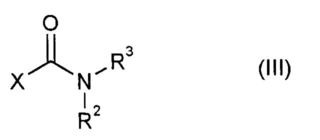 in welcher
   - R² und R³: die oben angegebene Bedeutung haben und
   - X: für Halogen steht,
   in Gegenwart eines Säureakzeptors und in Gegenwart eines Verdünnungsmittels bei Temperaturen zwischen 0°C und 200°C umsetzt und das hierbei als Nebenprodukt gebildete O-Carbamoylierungsprodukt der Formel (Ia) - unten - entweder durch Erhitzen zum gewünschten Produkt der Formel (I) isomerisiert oder durch Hydrolyse in wasserlösliche und somit leicht abtrennbare Komponenten überführt,
   oder wenn man
(b) (isolierte) Tetrazolinon-Metallsalze der allgemeinen Formel (IIa) 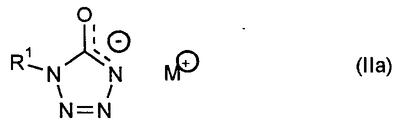 in welcher
   - R¹: die oben angegebene Bedeutung hat und
   - M: für ein Alkalimetall-, ein Erdalkalimetall- oder ein Erdmetalläquivalent steht,
   mit Carbamidsäurehalogeniden der allgemeinen Formel (III) 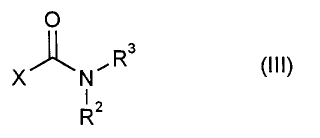 in welcher
   - R² und R³: die oben angegebene Bedeutung haben und
   - X: für Halogen steht,
   in Gegenwart eines Verdünnungsmittels bei Temperaturen zwischen 0°C und 200°C umsetzt und das hierbei als Nebenprodukt angefallene O-Carbamoylierungsprodukt der Formel (Ia) - unten - entweder durch Erhitzen zum gewünschten Produkt der Formel (I) isomerisiert oder durch Hydrolyse in wasserlösliche und somit leicht abtrennbare Komponenten überführt,
   oder wenn man
(c) substituierte Carbamoyloxytetrazole der allgemeinen Formel (Ia) 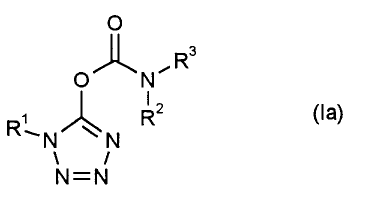 in welcher
   R¹, R² und R³ die oben angegebenen Bedeutungen haben,
   - welche auch in Mischung mit den Verbindungen der Formel (I) vorliegen können -
   gegebenenfalls in Gegenwart eines Verdünnungsmittels und gegebenenfalls in Gegenwart eines Reaktionshilfsmittels bei Temperaturen zwischen 50°C und 200°C zu den entsprechenden N-Carbamoyl-tetrazolinonen der Formel (I) isomerisiert,
   oder wenn man
(d) substituierte Carbamoyloxytetrazole der allgemeinen Formel (Ia) 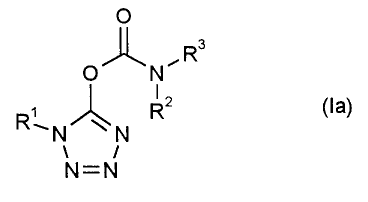 in welcher
   R¹, R² und R³ die oben angegebenen Bedeutungen haben,
   - welche als Produkte der Umsetzung von Verbindungen der Formel (II) bzw. von Verbindungen der Formel (IIa) mit Verbindungen der Formel (III) in Mischung mit den Verbindungen der Formel (I) vorliegen -
   mit Wasser und/oder einem Alkohol, insbesondere Methanol, gegebenenfalls in Gegenwart eines inerten organischen Lösungsmittels und gegebenenfalls in Gegenwart eines Reaktionshilfsmittels bei Temperaturen zwischen 10°C und 150°C umsetzt und die Produkte der Hydrolyse bzw. Alkoholyse der Verbindungen der Formel (Ia) nach üblichen Methoden abtrennt.

Überraschenderweise können nach dem erfindungsgemäßen Verfahren in den oben unter (a), (b), (c) und (d) beschriebenen vier Varianten die N-Carbamoyltetrazolinone der Formel (I) in meist erheblich höheren Ausbeuten und in stark verbesserter Qualität im Vergleich mit dem bekannten Stand der Technik hergestellt werden, wobei insbesondere der Anteil der unerwünschten Carbamoyloxytetrazole der Formel (Ia) minimiert werden kann.

Das erfindungsgemäße Verfahren stellt somit in all seinen Varianten eine wertvolle Bereicherung des Standes der Technik dar.

Das erfindungsgemäße Verfahren betrifft vorzugsweise die Herstellung von Verbindungen der Formel (I), in welcher
- R¹: für gegebenenfalls durch Carboxy, Cyano, Carbamoyl, Halogen, C₁-C₆-Alkoxy, C₁-C₆-Alkylthio, C₁-C₆-Alkylsulfinyl oder C₁-C₆-Alkylsulfonyl substituiertes Alkyl mit 1 bis 10 Kohlenstoffatomen, für jeweils gegebenenfalls durch Carboxy, Cyano, Carbamoyl oder Halogen substituiertes Alkenyl oder Alkinyl mit jeweils 2 bis 10 Kohlenstoffatomen, für jeweils gegebenenfalls durch Carboxy, Cyano, Carbamoyl, Halogen, C₁-C₄-Alkyl oder C₁-C₄-Alkoxy-carbonyl substituiertes Cycloalkyl oder Cycloalkylalkyl mit jeweils 3 bis 8 Kohlenstoffatomen im Cycloalkylteil und gegebenenfalls 1 bis 4 Kohlenstoffatomen im Alkylteil, für jeweils gegebenenfalls durch Carboxy, Cyano, Carbamoyl, Nitro, Amino, Hydroxy, Halogen, durch C₁-C₄-Alkyl, C₁-C₄-Alkoxy, C₁-C₄-Alkylthio, C₁-C₄-Alkylsulfinyl, C₁-C₄-Alkylsulfonyl, Di-(C₁-C₄-alkyl)amino, C₁-C₄-Alkylsulfonylamino, Di-(C₁-C₄-alkyl)aminosulfonyl, C₁-C₄-Alkyl-carbonyl, C₁-C₄-Alkylcarbonylamino, C₁-C₄-Alkoxy-carbonyl, Di-(C₁-C₄-alkylamino)-carbonyl, C₁-C₄-Alkylendioxy, Phenyl oder Phenoxy (welche jeweils gegebenenfalls durch Fluor und/oder Chlor substituiert sind) substituiertes Phenyl, Naphthyl, Phenyl-C₁-C₄-alkyl, Naphthyl-C₁-C₄-alkyl, Furyl, Benzofuryl, Tetrahydrofuryl, Furylmethyl, Tetrahydrofurylmethyl, Thienyl, Benzothienyl, Tetrahydrothienyl, Thienylmethyl, Tetrahydrothienylmethyl, Pyrrolyl, Indolyl, Oxazolyl, Benzoxazolyl, Oxazolylmethyl, Isoxazolyl, Isoxazolylmethyl, Thiazolyl, Benzthiazolyl, Thiazolylmethyl, Pyrazolyl, Oxadiazolyl, Oxadiazolylmethyl, Thiadiazolyl, Thiadiazolylmethyl, Triazolyl, Pyridyl, Chinolinyl, Isochinolinyl, Pyridylmethyl, Pyrazinyl, Pyridazinyl, Pyrimidinyl, Chinazolinyl, Chinoxalinyl, Pyrimidinylmethyl, Triazinyl oder Triazinylmethyl steht,
- R²: für jeweils gegebenenfalls durch Cyano oder Halogen substituiertes Alkyl, Alkenyl oder Alkinyl mit jeweils bis zu 6 Kohlenstoffatomen steht und
- R³: für jeweils gegebenenfalls durch Cyano oder Halogen substituiertes Alkyl mit 1 bis 6 Kohlenstoffatomen, für für jeweils gegebenenfalls durch Cyano oder Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 2 Kohlenstoffatomen im Alkylteil, oder für jeweils gegebenenfalls durch Cyano, Nitro, Halogen, durch C₁-C₄-Alkyl, C₁-C₄-Alkoxy, C₁-C₄-Alkylthio, C₁-C₄-Alkylsulfinyl, C₁-C₄-Alkylsulfonyl, Di-(C₁-C₄-alkyl)amino, C₁-C₄-Alkylsulfonylamino, Di-(C₁-C₄-alkyl)aminosulfonyl, C₁-C₄-Alkyl-carbonyl oder C₁-C₄-Alkoxy-carbonyl (welche jeweils gegebenenfalls durch Fluor und/oder Chlor substituiert sind) substituiertes Phenyl oder Phenyl-C₁-C₂-alkyl steht,
oder zusammen mit R² für Alkandiyl mit 2 bis 6 Kohlenstoffatomen steht.

Das erfindungsgemäße Verfahren betrifft insbesondere die Herstellung von Verbindungen der Formel (I), in welcher
- R¹: für jeweils gegebenenfalls durch Cyano, Fluor, Chlor, Methoxy, Ethoxy, Methylthio, Ethylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, n-, i- oder s-Pentyl, n-, i- oder s-Hexyl, für jeweils gegebenenfalls durch Cyano, Fluor, Chlor oder Brom substituiertes Propenyl, Butenyl, Pentenyl, Hexenyl, Propinyl, Butinyl, Pentinyl oder Hexinyl, für jeweils gegebenenfalls durch Cyano, Fluor, Chlor, Brom substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls durch Cyano, Fluor, Chlor, Brom, durch Methyl, Ethyl, n- oder i-Propyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Dimethylaminosulfonyl, Diethylaminosulfonyl, Methylcarbonyl, Ethylcarbonyl, n- oder i-Propylcarbonyl, Methoxycarbonyl, Ethoxycarbonyl, n- oder i-Propoxycarbonyl, Dimethylaminocarbonyl, Diethylaminocarbonyl, Methylendioxy oder Ethylendioxy (welche jeweils gegebenenfalls durch Fluor und/oder Chlor substituiert sind) substituiertes Phenyl, Benzyl, Phenylethyl, Furyl, Tetrahydrofuryl, Furylmethyl, Tetrahydrofurylmethyl, Thienyl, Tetrahydrothienyl, Thienylmethyl, Tetrahydrothienylmethyl, Oxazolyl, Oxazolylmethyl, Isoxazolyl, Isoazolylmethyl, Thiazolyl, Thiazolylmethyl, Oxadiazolyl, Oxadiazolylmethyl, Thiadiazolyl, Thiadiazolylmethyl, Pyridyl, Pyridylmethyl, Pyrimidinyl, Pyrimidinylmethyl, Triazinyl oder Triazinylmethyl steht,
- R²: für jeweils gegebenenfalls durch Cyano, Fluor oder Chlor substituiertes Methyl, Ethyl, n- oder i-Propyl, n-, i- oder s-Butyl, Propenyl, Butenyl, Propinyl oder Butinyl steht und
- R³: für jeweils gegebenenfalls durch Cyano, Fluor oder Chlor substituiertes Methyl, Ethyl, n- oder i-Propyl, n-, i- oder s-Butyl, Propenyl, Butenyl, Propinyl oder Butinyl, für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Cyano, Fluor, Chlor, Methyl, Ethyl, Methoxy oder Ethoxy substituiertes Phenyl, Benzyl oder Phenylethyl steht,
oder zusammen mit R² und dem damit verbundenen N-Atom für Pyrrolidino, Piperidino oder Morpholino steht.

Die bei der oben unter (a) beschriebenen Variante des erfindungsgemäßen Verfahrens - "Verfahrensvariante (a)" - als Ausgangsstoffe zu verwendenden Tetrazolinone sind durch die Formel (II) allgemein definiert. R¹ hat bei den Verbindungen der Formel (II) vorzugsweise bzw. insbesondere diejenige Bedeutung, die bereits oben bei der Beschreibung der nach dem erfindungsgemäßen Verfahren herzustellenden Verbindungen der Formel (I) als bevorzugt bzw. als insbesondere bevorzugt für R¹ angegeben wurde.

Die Ausgangsstoffe der Formel (II) sind bekannt und/oder können nach an sich bekannten Verfahren hergestellt werden (vgl. J. Am. Chem. Soc. 81 (1959), 3076-3079; J. Org. Chem. 45 (1980), 5130-5136; EP-A 146279; EP-A 572855; EP-A 578090).

Die bei der Verfahrensvariante (a) sowie bei der Verfahrensvariante (b) als Ausgangsstoffe zu verwendenden Carbamidsäurehalogenide sind durch die Formel (III) allgemein definiert. R² und R³ haben bei den Verbindungen der Formel (III) vorzugsweise bzw. insbesondere diejenige Bedeutung, die bereits oben bei der Beschreibung der nach dem erfindungsgemäßen Verfahren herzustellenden Verbindungen der Formel (I) als bevorzugt bzw. als insbesondere bevorzugt für R² bzw. R³ angegeben wurde; X steht vorzugsweise für Fluor, Chlor oder Brom, insbesondere für Chlor.

Die Ausgangsstoffe der Formel (III) sind bekannte organische Synthesechemikalien.

Die bei der Verfahrensvariante (b) als Ausgangsstoffe zu verwendenden Tetrazolinon-Metallsalze sind durch die Formel (IIa) allgemein definiert. R¹ hat bei den Verbindungen der Formel (II) vorzugsweise bzw. insbesondere diejenige Bedeutung, die bereits oben bei der Beschreibung der nach dem erfindungsgemäßen Verfahren herzustellenden Verbindungen der Formel (I) vorzugsweise bzw. als insbesondere bevorzugt für R¹ angegeben wurde; M steht vorzugsweise für Lithium, Natrium oder Kalium oder für ein Magnesium-, Calcium-, Barium- oder Aluminiumäquivalent, insbesondere für Natrium oder Kalium.

Die Tetrazolinon-Metallsalze der Formel (IIa) sind als isolierte Produkte noch nicht aus der Literatur bekannt; sie sind als neue Stoffe auch Gegenstand der vorliegenden Anmeldung.

Man erhält die neuen Tetrazolinon-Metallsalze der Formel (IIa), wenn man Tetrazolinone der allgemeinen Formel (II) 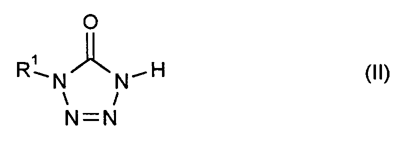 in welcher
- R¹: die oben angegebene Bedeutung hat,
mit Alkalimetall-, Erdalkalimetall- oder Erdmetall-verbindungen der allgemeinen Formel (IV)

M-Z (IV)

in welcher
- M: für ein Alkalimetall-, Erdalkalimetall- oder ein Erdmetall-äquivalent, vorzugsweise für ein Lithium-, Natrium-, Kalium-, Magnesium-, Calcium-, Barium- oder Aluminium-äquivalent, insbesondere für Natrium oder Kalium, steht und
- Z: für Hydroxy, ein Carbonat- bzw. ein Hydrogencarbonat-äquivalent oder - vorzugsweise - ein Alkoholat-äquivalent, vorzugsweise für Methoxy, Ethoxy, n- oder i-Propoxy, n-, i-, s- oder t-Butoxy, insbesondere für Methoxy oder Ethoxy steht,
in Gegenwart eines Verdünnungsmittels, wie z.B. Methanol, Ethanol, n- oder i-Propanol, n-, i-, s- oder t-Butanol, bei Temperaturen zwischen 0°C und 150°C, vorzugsweise zwischen 20°C und 120°C umsetzt und dann das Verdünnungsmittel - vorzugsweise unter vermindertem Druck - abdestilliert.

Die bei den Verfahrensvarianten (c) und (d) als Ausgangsstoffe verwendeten substituierten Carbamoyloxytetrazole sind durch die Formel (Ia) allgemein definiert. In der Formel (Ia) haben R¹, R² und R³ vorzugsweise bzw. insbesondere diejenige Bedeutung, die bereits oben bei der Beschreibung der nach dem erfindungsgemäßen Verfahren herzustellenden Verbindungen der Formel (I) vorzugsweise bzw. als insbesondere bevorzugt für R¹, R² bzw. R³ angegeben wurde.

Die Carbamoyloxytetrazole der Formel (Ia) sind bisher noch nicht aus der Literatur bekannt; sie sind als neue Stoffe ebenfalls Gegenstand der vorliegenden Anmeldung. In gewissem Umfang zeigen die Verbindungen der Formel (Ia) auch herbizide Wirksamkeit.

Die Carbamoyloxytetrazole der Formel (Ia) fallen im allgemeinen bei der Umsetzung von Tetrazolinonen der Formel (II) oder ihren Salzen mit Carbamidsäurechloriden der Formel (III) bei niedrigen oder mäßig erhöhten Temperaturen in Gemischen mit N-Carbamoyl-tetrazolinonen der Formel (I) an. Sie können in diesen Gemischen gemäß den Verfahrensvarianten (c) und (d) umgesetzt werden, aber auch vorher nach üblichen Methoden aus diesen Gemischen isoliert werden.

Die erfindungsgemäße Verfahrensvariante (a) wird in Gegenwart eines geeigneten Säureakzeptors durchgeführt. Als solche kommen alle üblichen anorganischen oder organischen Basen infrage. Hierzu gehören beispielsweise Alkalimetall- oder Erd-alkalimetall- -hydride, -hydroxide, -amide, -alkoholate, -acetate, -carbonate oder - hydrogencarbonate, wie beispielsweise Lithium-, Natrium-, Kalium- oder Calcium-hydrid, Lithium-, Natrium- oder Kalium-amid, Natrium- oder Kalium-methylat, Natrium- oder Kalium-ethylat, Natrium- oder Kalium-propylat, Aluminiumisopropylat, Natrium- oder Kalium-tert-butylat, Natrium- oder Kalium-hydroxid, Natrium-, Kalium- oder Calcium-acetat, Natrium-, Kalium- oder Calcium-carbonat, Natrium- oder Kalium-hydrogencarbonat, sowie basische organische Stickstoffverbindungen, wie Trimethylamin, Triethylamin, Tripropylamin, Tributylamin, Ethyl-diisopropylamin, N,N-Dimethylcyclohexylamin, Dicyclohexylamin, Ethyl-dicyclohexylamin, N,N-Dimethylanilin, N,N-Dimethyl-benzylamin, Pyridin, 2-Methyl-, 3-Methyl- und 4-Methyl-pyridin, 2,4-Dimethyl-, 2,6-Dimethyl-, 3,4-Dimethyl- und 3,5-Dimethyl-pyridin, 5-Ethyl-2-methyl-pyridin, N-Methylpiperidin, 4-Dimethylaminopyridin, Diazabicyclooctan (DABCO), Diazabicyclononen (DBN) oder Diazabicycloundecen (DBU).

Vorzugsweise werden Lithium-, Natrium-, Kalium- oder Calcium-carbonat bzw. die entsprechenden Hydrogencarbonate als Säureakzeptoren bei der erfindungsgemäßen Verfahrensvariante (a) eingesetzt.

Als Verdünnungsmittel zur Durchführung der erfindungsgemäßen Verfahrensvarianten (a) kommen die üblichen inerten organischen Lösungsmittel in Betracht. Hierzu gehören insbesondere aliphatische, alicyclische oder aromatische, gegebenenfalls halogenierte Kohlenwasserstoffe, wie beispielsweise Pentan, Hexan, Heptan, Petrolether, Ligroin, Benzin, Benzol, Toluol, Xylol, Chlorbenzol, Dichlorbenzol, Cyclohexan, Methylcyclohexan, Dichlormethan, Chloroform, Tetrachlormethan; Ether, wie Diethylether, Diisopropylether, t-Butyl-methylether, t-Pentyl-methylether, Dioxan, Tetrahydrofuran, Ethylenglykol-dimethyl- oder -diethylether, Diethylenglykol-dimethylether oder -diethylether; Ketone, wie Aceton, Butanon oder Methyl-isobutyl-keton; Nitrile, wie Acetonitril, Propionitril, Butyronitril oder Benzonitril; Amide, wie N,N-Dimethylformamid, N,N-Dimethylacetamid, N-Methylformanilid, N-Methyl-pyrrolidon oder Hexamethylphosphorsäuretriamid; Ester wie Essigsäure-methylester, -ethylester, -n- oder -i-propylester, -n-, -i- oder - s-butylester; Sulfoxide, wie Dimethylsulfoxid; Alkohole, wie Methanol, Ethanol, n- oder i-Propanol, n-, i-, s- oder t-Butanol, Ethylenglykol-monomethylether oder - monoethylether, Diethylenglykolmonomethylether oder -monoethylether.

Vorzugsweise werden aprotisch polare Lösungsmittel, wie z.B. t-Pentyl-methylether, Ethylenglykol-dimethylether, Ethylenglykol-diethylether, Methylisobutyl-keton, Acetonitril, Propionitril, Butyronitril, Essigsäure-ethylester, Essigsäure-propylester und Essigsäure-butylester als Verdünnungsmittel bei der erfindungsgemäßen Verfahrensvariante (a) eingesetzt.

Die Reaktionstemperaturen können bei der Durchführung der erfindungsgemäßen Verfahrensvariante (a) in einem größeren Bereich variiert werden. Im allgemeinen arbeitet man bei Temperaturen zwischen 0°C und 200°C, in der Anfangsphase vorzugsweise bei Temperaturen zwischen 10°C und 80°C, insbesondere zwischen 20°C und 60°C, anschließend vorzugsweise zwischen 50°C und 150°C, insbesondere zwischen 70°C und 130°C.

Die erfindungsgemäße Verfahrensvariante (a) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung der erfindungsgemäßen Verfahrensvariante (a) zur Herstellung der Verbindungen der Formel (I) setzt man pro Mol Tetrazolinon der Formel (II) im allgemeinen 0,9 bis 1,5 Mol, vorzugsweise 1,0 bis 1,2 Mol, Carbamidsäurehalogenid der Formel (III) und 1,0 bis 1,5 Mol, vorzugsweise 1,0 bis 1,2 Mol, Säureakzeptor ein.

In einer bevorzugten Ausführungsform der erfindungsgemäßen Verfahrensvariante (a) wird ein Tetrazolinon der Formel (II) mit einem Säureakzeptor und einem Verdünnungsmittel im unteren Bereich des oben angegebenen Temperaturintervalls vermischt und das Carbamidsäurehalogenid der Formel (III) wird dazu gegeben. Dann wird die Reaktionsmischung bis zum Ende der Umsetzung bzw. Umlagerung (im allgemeinen eine oder mehrere Stunden, meist etwa 1 bis 25 Stunden) im oberen Bereich des oben angegebenen Temperaturintervalls gehalten.

Die weiteren Schritte bis zur Isolierung der Produkte der Formel (I) können zweckmäßigerweise wie folgt durchgeführt werden:
Nach Abkühlen wird mit Wasser versetzt, die organische Phase abgetrennt, gegebenenfalls nachextrahiert und von den vereinigten organischen Phasen das Lösungsmittel abdestilliert. Der Rückstand wird mit Wasser und gegebenenfalls einem organischen Lösungsmittel, wie z.B. Methanol oder Toluol, gegebenenfalls in Gegenwart eines Reaktionshilfsmittels, wie z.B. Salzsäure, mehrere Stunden erhitzt. Dann wird - gegebenenfalls nach Zugabe eines mit Wasser praktisch nicht mischbaren organischen Lösungsmittels, wie z.B. Toluol - die organische Phase abgetrennt, gegebenenfalls nachextrahiert, die vereinigten organischen Phasen werden mit verdünnter wässriger Natronlauge verrührt, dann wird abgetrennt, getrocknet und filtriert. Nach Einengen wird der Rückstand durch Digerieren mit einem geeigneten organischen Lösungsmittel, wie z.B. Hexan, zur Kristallisation gebracht und das Produkt wird durch Absaugen isoliert.

Die erfindungsgemäße Verfahrensvariante (b) wird in Gegenwart eines Verdünnungsmittels durchgeführt. Es kommen hierbei im wesentlichen die gleichen Verdünnungsmittel in Betracht, die oben für die erfindungsgemäße Verfahrensvariante (a) genannt worden sind.

Vorzugsweise werden aprotisch polare Lösungsmittel, wie z.B. Ethylenglykol-dimethylether, Ethylenglykol-diethylether, Methylisobutyl-keton, Acetonitril, Propionitril, Butyronitril, Essigsäure-ethylester, Essigsäure-propylester und Essigssäure-butylester als Verdünnungsmittel auch bei der erfindungsgemäßen Verfahrensvariante (b) eingesetzt.

Die Reaktionstemperaturen können bei der Durchführung der erfindungsgemäßen Verfahrensvariante (b) in einem größeren Bereich variiert werden. Im allgemeinen arbeitet man bei Temperaturen zwischen 0°C und 200°C, in der Anfangsphase vorzugsweise bei Temperaturen zwischen 10°C und 80°C, insbesondere zwischen 20°C und 60°C, anschließend vorzugsweise zwischen 50°C und 150°C, insbesondere zwischen 70°C und 130°C.

Die erfindungsgemäße Verfahrensvariante (b) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung der erfindungsgemäßen Verfahrensvariante (b) zur Herstellung der Verbindungen der Formel (I) setzt man pro Mol Tetrazolinon-Metallsalz der Formel (IIa) im allgemeinen 0,9 bis 1,5 Mol, vorzugsweise 1,0 bis 1,2 Mol Carbamidsäurehalogenid der Formel (III) ein.

In einer bevorzugten Ausführungsform der erfindungsgemäßen Verfahrensvariante (b) wird in einer vorgeschalteten Stufe ein Tetrazolinon-Metallsalz der Formel (IIa) aus einem Tetrazolinon der Formel (II) und einer Alkalimetall-, Erdalkalimetall- oder Erdmetall-verbindung der Formel (IV) hergestellt und durch Abdestillieren des Lösungsmittels isoliert. Das so erhaltene Produkt wird dann in einem der obengenannten inerten Verdünnungsmittel aufgenommen und mit einem Carbamidsäurehalogenid der Formel (III) versetzt; die Mischung wird dann noch einige Zeit (im allgemeinen etwa 1 bis 25 Stunden) im oberen Bereich des oben angegebenen Temperaturintervalls gehalten.

Die Aufarbeitung kann nach üblichen Methoden, beispielweise wie oben für die Verfahrensvariante (a) beschrieben, durchgeführt werden.

Die erfindungsgemäße Verfahrensvariante (c) wird gegebenenfalls in Gegenwart eines Verdünnungsmittels durchgeführt. Es kommen hierbei im wesentlichen die gleichen Verdünnungsmittel in Betracht, die oben für die erfindungsgemäße Verfahrensvariante (a) genannt worden sind. Die Verfahrensvariante (c) kann jedoch vorteilhaft auch ohne Verwendung eines Verdünnungsmittels durchgeführt werden.

Die Verfahrensvariante (c) wird gegebenenfalls in Gegenwart eines Reaktionshilfsmittels durchgeführt. Vorzugsweise kommen als Reaktionshilfsmittel hierbei basische organische Stickstoffverbindungen wie z.B. Trimethylamin, Triethylamin, Tripropylamin, Tributylamin, Ethyl-diisopropylamin, N,N-Dimethylcyclohexylamin, Dicyclohexylamin, Ethyl-dicyclohexylamin, N,N-Dimethylanilin, N,N-Dimethyl-benzylamin, Pyridin, 2-Methyl-, 3-Methyl- und 4-Methyl-pyridin, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methylpiperidin, N,N-Dimethylaminopyridin, Diazabicyclooctan (DABCO), Diazabicyclononen (DBN) oder Diazabicycloundecen (DBU) in Betracht. Insbesondere Pyridin und 4-Dimethylamino-pyridin sind als Reaktionshilfsmittel bei der Verfahrensvariante (c) geeignet. Es kommen neben oder an Stelle dieser Verbindungen auch Iod oder Alkalimetall-iodide als Reaktionshilfsmittel in Betracht.

Die Reaktionstemperaturen können bei der Durchführung der erfindungsgemäßen Verfahrensvariante (c) in einem größeren Bereich variiert werden. Im allgemeinen arbeitet man bei Temperaturen zwischen 50°C und 200°C, vorzugsweise zwischen 60°C und 170°C, insbesondere zwischen 80°C und 150°C.

Die erfindungsgemäße Verfahrensvariante (c) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung der Verfahrensvariante (c) werden die Reaktionskomponenten im allgemeinen bei Raumtemperatur vermischt und auf die erforderliche Reaktionstemperatur aufgeheizt, bis die Umsetzung (Isomerisierung) zum Stillstand gekommen ist. Dann kann nach der oben beschriebenen Methode (vgl. die Varianten (a) und (b)) aufgearbeitet und das Produkt durch Umkristallisation gereinigt werden.

Die erfindungsgemäße Verfahrensvariante (d) wird vorzugsweise in Gegenwart eines organischen Lösungsmittels durchgeführt. Es kommen hierbei im wesentlichen die oben bei der Beschreibung der Verfahrensvariante (a) aufgeführten Lösungsmittel in Betracht.

Die erfindungsgemäße Verfahrensvariante (d) wird vorzugsweise in Gegenwart eines geeigneten Reaktionshilfsmittels durchgeführt. Als solche kommen alle üblichen anorganischen oder organischen Basen infrage. Hierzu gehören beispielsweise Alkalimetall- oder Erdalkalimetall- -hydride, -hydroxide, -amide, -alkoholate, -acetate, -carbonate oder -hydrogencarbonate, wie beispielsweise Lithium-, Natrium-, Kalium- oder Calcium-hydrid, Lithium-, Natrium- oder Kalium-amid, Natrium- oder Kalium-methylat, Natrium- oder Kalium-ethylat, Natrium- oder Kalium-propylat, Aluminiumisopropylat, Natrium- oder Kalium-tert-butylat, Natrium- oder Kalium-hydroxid, Ammoniumhydroxid, Natrium-, Kalium- oder Calcium-acetat, Ammoniumacetat, Natrium-, Kalium- oder Calcium-carbonat, Ammoniumcarbonat, Natrium- oder Kalium-hydrogencarbonat, sowie basische organische Stickstoffverbindungen, wie Trimethylamin, Triethylamin, Tripropylamin, Tributylamin, Ethyl-diisopropylamin, N,N-Dimethylcyclohexylamin, Dicyclohexylamin, Ethyl-dicyclohexylamin, N,N-Dimethylanilin, N,N-Dimethylbenzylamin, Pyridin, 2-Methyl-, 3-Methyl- und 4-Methyl-pyridin, 2,4-Dimethyl-, 2,6-Dimethyl-, 3,4-Dimethyl- und 3,5-Dimethyl-pyridin, 5-Ethyl-2-methyl-pyridin, N-Methylpiperidin, 4-Dimethylaminopyridin, Diazabicyclooctan (DABCO), Diazabicyclononen (DBN) oder Diazabicycloundecen (DBU).

Als Reaktionshilfsmittel kommen bei der Verfahrensvariante (d) alternativ auch Säuren in Betracht. Hierzu gehören anorganische Säuren oder Mineralsäuren, wie z.B. Salzsäure, Schwefelsäure, Phosphorsäure und Salpetersäure, aber auch organische Säuren, wie z.B. Essigsäure, Methansulfonsäure, Benzolsulfonsäure und p-Toluolsulfonsäure.

Die erfindungsgemäße Verfahrensvariante (d) kann gegebenenfalls auch in einem Zweiphasensystem, wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutyl-ammoniumiodid, Tetrabutylammoniumbromid, Tetrabutylammoniumchlorid, Tributyl-methylphosphoniumbromid, Trimethyl-C₁₃/C₁₅-alkylammoniumchlorid, Trimethyl-C₁₃/C₁₅-alkylammoniumbromid, Dibenzyl-dimethyl-ammoniummethylsulfat, Dimethyl-C₁₂/C₁₄-alkyl-benzylammoniumchlorid, Dimethyl-C₁₂/C₁₄-alkyl-benzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Die Reaktionstemperaturen können bei der Durchführung der erfindungsgemäßen Verfahrensvariante (d) in einem größeren Bereich variiert werden. Im allgemeinen arbeitet man bei Temperaturen zwischen 10°C und 150°C, vorzugsweise bei Temperaturen zwischen 20°C und 120°C, insbesondere zwischen 25°C und 100°C.

Die erfindungsgemäße Verfahrensvariante (d) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung der Verfahrensvariante (d) werden die Reaktionskomponenten im allgemeinen bei Raumtemperatur vermischt und auf die erforderliche Reaktionstemperatur aufgeheizt, bis die Umsetzung (Hydrolyse oder Alkoholyse) abgeschlossen ist. Dann kann nach üblichen Methoden (vgl. die Varianten (a) und (b)) aufgearbeitet werden.

Die erfindungsgemäß herstellbaren substituierten N-Carbamoyl-tetrazolinone der Formel (I) können als Herbizide zur Bekämpfung unerwünschten Pflanzenwuchses verwendet werden (vgl. EP-A 146 279, EP-A 202 929, EP-A 571 854, EP-A 571 855, EP-A 572 855, EP-A 578 090 und EP-A 612 735).

### Herstellungsbeispiele:

### Beispiel 1

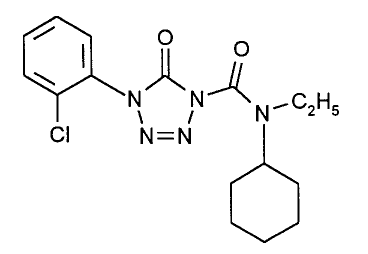

### (A) Herstellung einer Verbindung der Formel (IIa):

Eine Mischung aus 39,3 g (200 mMol) 1-(2-Chlor-phenyl)-1,4-dihydro-5H-tetrazol-5-on, 11,0 g (201 mMol) Natriummethylat und 200 ml Methanol wird 4 Stunden unter Rückfluß erhitzt. Dann wird das Lösungsmittel im Wasserstrahlvakuum sorgfältig abdestilliert. Man erhält das Natriumsalz von 1-(2-Chlor-phenyl)-1,4-dihydro-5H-tetrazol-5-on in quantitativer Ausbeute als festen Rückstand (Schmelzpunkt: 170°C unter Zersetzung), der im gleichen Reaktionsgefäß weiter umgesetzt werden kann.

### (B) Herstellung einer Verbindung der Formel (I) nach Verfahrensvariante (b):

10,7 g (50 mMol) Natriumsalz von 1-(2-Chlor-phenyl)-1,4-dihydro-5H-tetrazol-5-on werden in 100 ml 1,2-Dimethoxyethan vorgelegt und bei Raumtemperatur (ca. 20°C) mit einer Lösung von 10,0 g (52,5 mMol) N-Cyclohexyl-N-ethyl-carbamidsäurechlorid in 10 ml 1,2-Dimethoxyethan versetzt. Die Mischung wird dann ca. 17 Stunden unter Rückfluß erhitzt. Anschließend wird eingeengt, der Rückstand mit Essigsäureethylester/Wasser geschüttelt, die organische Phase abgetrennt, die wässrige Phase mit Essigsäureethylester nachextrahiert, die vereinigten organischen Phasen werden mit Natriumsulfat getrocknet und filtriert. Das Filtrat wird eingeengt, der Rückstand mit einem Gemisch aus 200 ml Methanol und 50 ml Wasser 17 Stunden unter Rückfluß erhitzt und erneut eingeengt. Der Rückstand wird in Essigsäureethylester aufgenommen und mit 50 ml 1N-Natronlauge verrührt. Dann werden die Phasen getrennt, die wässrige Phase wird mit Toluol nachextrahiert, die organischen Phasen werden vereinigt, mit Natriumsulfat getrocknet und filtriert. Das Filtrat wird eingeengt und der Rückstand aus Hexan umkristallisiert.

Man erhält 15,4 g (88% der Theorie) 1-(2-Chlor-phenyl)-4-(N-cyclohexyl-N-ethyl-aminocarbonyl)-1,4-dihydro-5H-tetrazol-5-on.
Das entsprechende (isomere) O-Carbamoylierungsprodukt ist dabei mit üblichen analytischen Methoden nicht mehr nachweisbar.

¹⁵N-NMR (D7-DMF, ext.Referenz: Nitromethan): -25,7, -32,2, -162,5, -176,4, -249 ppm.

### Beispiel 2

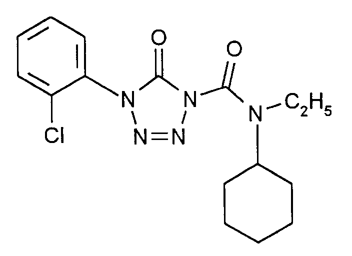

### (A) Herstellung einer Verbindung der Formel (IIa):

Eine Mischung aus 9,8 g (50 mMol) 1-(2-Chlor-phenyl)-1,4-dihydro-5H-tetrazol-5-on, 2,8 g (51 mMol) Natriummethylat und 100 ml Methanol wird 2 Stunden unter Rückfluß erhitzt. Dann wird das Lösungsmittel im Wasserstrahlvakuum sorgfältig abdestilliert. Man erhält das Natriumsalz von 1-(2-Chlor-phenyl)-1,4-dihydro-5H-tetrazol-5-on in quantitativer Ausbeute als festen Rückstand, der im gleichen Reaktionsgefäß weiter umgesetzt werden kann.

### (B) Herstellung einer Verbindung der Formel (I) nach Verfahrensvariante (b):

Die komplette Menge des gemäß obiger Beschreibung erhaltenen Natriumsalzes von 1-(2-Chlor-phenyl)-1,4-dihydro-5H-tetrazol-5-on wird in 100 ml 1,2-Dimethoxyethan vorgelegt und bei Raumtemperatur (ca. 20°C) mit einer Lösung von 10,4 g (55 mMol) N-Cyclohexyl-N-ethyl-carbamidsäurechlorid in 10 ml 1,2-Dimethoxyethan versetzt. Die Mischung wird dann ca. 17 Stunden unter Rückfluß erhitzt. Anschließend wird eingeengt, der Rückstand mit einem Gemisch aus 200 ml Methanol und 50 ml Wasser 9 Stunden unter Rückfluß erhitzt und erneut eingeengt. Der Rückstand wird in Toluol aufgenommen und mit 50 ml 1N-Natronlauge verführt. Dann werden die Phasen getrennt, die wässrige Phase wird mit Toluol nachextrahiert, die organischen Phasen werden vereinigt, mit Natriumsulfat getrocknet und filtriert. Das Filtrat wird eingeengt und der Rückstand aus Hexan umkristallisiert.

Man erhält 15,2 g (87% der Theorie) 1-(2-Chlor-phenyl)-4-(N-cyclohexyl-N-ethyl-aminocarbonyl)-1,4-dihydro-5H-tetrazol-5-on.
Das entsprechende (isomere) O-Carbamoylierungsprodukt ist dabei mit üblichen analytischen Methoden nicht mehr nachweisbar.

### Beispiel 3

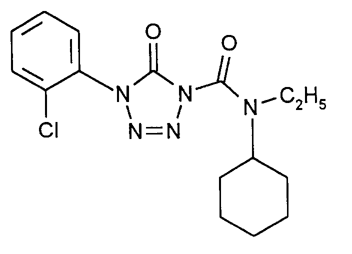

(nach Verfahrensvariante (c)):

11,6 mg einer Mischung aus 1-(2-Chlor-phenyl)-4-(N-cyclohexyl-N-ethyl-aminocarbonyl)-1,4-dihydro-5H-tetrazol-5-on und dem entsprechenden isomeren Carbamoyloxytetrazol im Gewichtsverhältnis 57:37 wird langsam auf ca. 170°C aufgeheizt. Nach Erreichen dieser Temperatur wird das Erhitzen abgebrochen und eine Gehaltsbestimmung durchgeführt. Es werden 89% 1-(2-Chlor-phenyl)-4-(N-cyclohexyl-N-ethyl-aminocarbonyl)-1,4-dihydro-5H-tetrazol-5-on und nur noch 2% des entsprechenden isomeren Carbamoyloxytetrazols gefunden. Daneben werden 2 Komponenten unbekannter Struktur mit jeweils 6%-Anteil gefunden.

Wenn die oben beschriebene pyrolytische Isomerisierung nach Zugabe von 0,01 ml Pyridin durchgeführt wird, werden 94% 1-(2-Chlor-phenyl)-4-(N-cyclohexyl-N-ethyl-aminocarbonyl)-1,4-dihydro-5H-tetrazol-5-on und nur noch 4% einer unbekannten Nebenkomponente gefunden, jedoch kein isomeres Carbamoyloxytetrazol.

### Beispiel 4

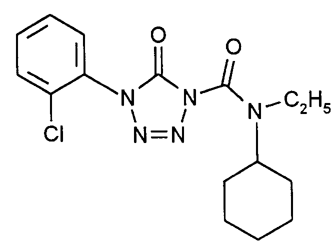

(nach Verfahrensvariante (a)):

2,0 g einer Mischung aus 1-(2-Chlor-phenyl)-4-(N-cyclohexyl-N-ethyl-aminocarbonyl)-1,4-dihydro-5H-tetrazol-5-on und dem entsprechenden isomeren Carbamoyloxytetrazol im Gewichtsverhältnis 1:1 werden in einer Mischung aus 80 ml Methanol und 20 ml Wasser 20 Stunden unter Rückfluß erhitzt. Dann wird eingeengt, der Rückstand in Methylenchlorid aufgenommen, mit 20 ml 1N-Natronlauge geschüttelt, die wässrige Phase mit Methylenchlorid nachextrahiert, die vereinigten organischen Phasen werden mit Natriumsulfat getrocknet und filtriert. Vom Filtrat wird das Lösungsmittel im Wasserstrahlvakuum sorgfältig abdestilliert.
Man erhält 1-(2-Chlor-phenyl)-4-(N-cyclohexyl-N-ethyl-aminocarbonyl)-1,4-dihydro-5H-tetrazol-5-on als kristallinen Rückstand in quantitativer Ausbeute.

### Beispiel 5

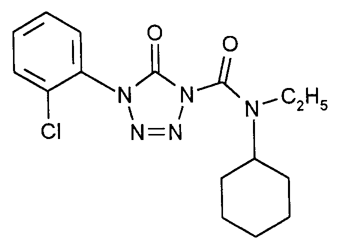

(nach Verfahrensvariante (d)):

1,0 g einer Mischung aus 1-(2-Chlor-phenyl)-4-(N-cyclohexyl-N-ethyl-aminocarbonyl)-1,4-dihydro-5H-tetrazol-5-on und dem entsprechenden isomeren Carbamoyloxytetrazol im Gewichtsverhältnis 1:1 werden in einer Mischung aus 10 ml Toluol und 10 ml 10%iger wässriger Salzsäure 17 Stunden bei ca. 20°C gerührt. Dann wird die organische Phase abgetrennt, mit 10 ml 1N-Natronlauge geschüttelt, die wässrige Phase wird mit Methylenchlorid nachextrahiert, die vereinigten organischen Phasen werden mit Natriumsulfat getrocknet und filtriert. Vom Filtrat wird das Lösungsmittel im Wasserstrahlvakuum sorgfältig abdestilliert.

Man erhält 1-(2-Chlor-phenyl)-4-(N-cyclohexyl-N-ethyl-aminocarbonyl)-1,4-dihydro-5H-tetrazol-5-on als kristallinen Rückstand in quantitativer Ausbeute.

### Beispiel 6

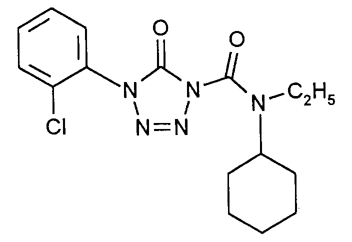

(nach Verfahrensvariante (a)):

9,8 g (50 mMol) 1-(2-Chlor-phenyl)-1,4-dihydro-5H-tetrazol-5-on werden mit 5,8 g (54 mMol) Natriumcarbonat in 100 ml Essigsäureethylester vorgelegt und mit 10,4 g (54 mMol) N-Cyclohexyl-N-ethyl-carbamidsäurechlorid in 20 ml Essigsäureethylester versetzt. Die Mischung wird 16 Stunden unter Rückfluß erhitzt und nach Abkühlen auf Raumtemperatur (ca. 20°C) mit Wasser geschüttelt. Die wässrige Phase wird dann mit Essigsäureethylester nachextrahiert. Dann werden die organischen Phasen vereinigt und im Wasserstrahlvakuum eingeengt. Der Rückstand wird mit 200 ml Toluol und 100 ml 10%iger wässriger Salzsäure 16 Stunden bei 20°C gerührt und nach Abtrennen der organischen Phase wird die wässrige Phase mit Toluol nachextrahiert. Die vereinigten organischen Phasen werden dann mit 100 ml 1N-Natronlauge 30 Minuten lang gerührt. Dann wird die organische Phase abgetrennt und die wässrige Phase wird mit Toluol nachextrahiert. Die vereinigten organischen Phasen werden mit Natriumsulfat getrocknet und filtriert. Das Filtrat wird im Wasserstrahlvakuum eingeengt und der Rückstand wird durch Digerieren mit n-Hexan zur Kristallisation gebracht.

Man erhält 16,0 g (Gehalt: 96,4%, Ausbeute: 91,5% der Theorie) 1-(2-Chlor-phenyl)-4-(N-cyclohexyl-N-ethyl-aminocarbonyl)-1,4-dihydro-5H-tetrazol-5-on.
Das entsprechende (isomere) O-Carbamoylierungsprodukt ist dabei mit üblichen analytischen Methoden nicht mehr nachweisbar.

### Beispiel 7

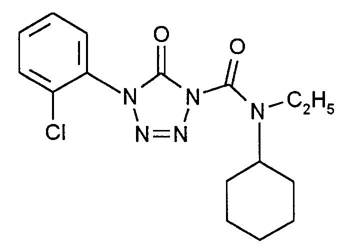

(nach Verfahrensvariante (a)):

9,8 g (50 mMol) 1-(2-Chlor-phenyl)-1,4-dihydro-5H-tetrazol-5-on werden mit 5,8 g (54 mMol) Natriumcarbonat in 100 ml Essigsäureethylester vorgelegt und mit 10,4 g (54 mMol) N-Cyclohexyl-N-ethyl-carbamidsäurechlorid in 20 ml Essigsäureethylester versetzt. Die Mischung wird 16 Stunden unter Rückfluß erhitzt und nach Abkühlen auf Raumtemperatur (ca. 20°C) mit Wasser geschüttelt. Die wässrige Phase wird dann mit Essigsäureethylester nachextrahiert. Dann werden die organischen Phasen vereinigt und im Wasserstrahlvakuum eingeengt. Der Rückstand wird mit 200 ml Methanol und 50 ml Wasser ca. 20 Stunden unter Rückfluß erhitzt und dann eingeengt. Der Rückstand wird in Toluol aufgenommen und mit 100 ml 1N-Natronlauge verrührt. Die weitere Aufarbeitung erfolgt wie in Beispiel 6 beschrieben.

Man erhält 15,2 g (Gehalt: 96,4%, Ausbeute: 87% der Theorie) 1-(2-Chlor-phenyl)-4-(N-cyclohexyl-N-ethyl-aminocarbonyl)-1,4-dihydro-5H-tetrazol-5-on.

### Carbamoyloxytetrazole der Formel (Ia):

### Beispiel (Ia-1)

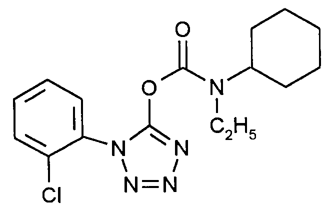

19,6 g (100 mMol) 1-(2-Chlor-phenyl)-1,4-dihydro-5H-tetrazol-5-on, 12,8 g (120 mMol) Natriumcarbonat und 0,1 g 4-Dimethylamino-pyridin werden in 150 ml Toluol vorgelegt und mit einer Lösung von 22,8 g (120 mMol) N-Cyclohexyl-N-ethyl-carbamidsäurechlorid in 50 ml Toluol versetzt. Die Mischung wird 17 Stunden bei 50°C bis 55°C gerührt und nach Abkühlen auf Raumtemperatur (ca. 20°C) mit Wasser geschüttelt. Die wässrige Phase wird dann mit Essigsäureethylester nachextrahiert. Dann werden die organischen Phasen vereinigt und im Wasserstrahlvakuum eingeengt.

Man erhält 34 g (89% der Theorie) eines 1:1-Gemisches aus 1-(2-Chlor-phenyl)-4-(N-cyclohexyl-N-ethyl-aminocarbonyl)-1,4-dihydro-5H-tetrazol-5-on und dem entsprechenden isomeren Carbamoyloxytetrazol.

Die Auftrennung in die beiden Isomeren erfolgt nach üblichen Methoden. So erhält man das Carbamoyloxytetrazol obiger Strukturformel beispielsweise chromatographisch (präparative HPLC: Nucleosil 100 CN, 5 µm, 250x4 mm; Heptan/t-Butyl-methylether Vol. 80/20, isokratisch 2 ml/min 3,5 µl injiziert 0,3%ige Lösung in Eluent) oder durch eine sog. Craig-Verteilung (200 Stufen, Essigsäureethylester/Dimethylformamid Vol. 3/7 und Heptan/Wasser Vol 7/3).

¹⁵N-NMR (D7-DMF, ext.Referenz: Nitromethan): +11,9, -8,2, -73,0, -156,3, -271,1 ppm.

### Anmerkung zur Nomenklatur:

Die in den Herstellungsbeispielen 1 bis 7 beschriebene Verbindung gemäß Formel (I) kann alternativ auch wie folgt bezeichnet werden:
- nach CHEMICAL ABSTRACTS:
   "4-(2-Chlorphenyl)-4,5-dihydro-N-cyclohexyl-N-ethyl-5-oxo-1H-tetrazol-1-carboxamid";
- nach den oben zum Stand der Technik zitierten Dokumenten:
   "1-(2-Chlorphenyl)-4-(N-cyclohexyl-N-ethyl-carbamoyl)-5(4H)-tetrazolinon".

Analoges gilt für die alternative Bezeichnung des verwendeten Ausgangsprodukts gemäß Formel (II):
- nach CHEMICAL ABSTRACTS:
   "4-(2-Chlorphenyl)-4,5-dihydro-5-oxo-1H-tetrazol";
- nach den oben zum Stand der Technik zitierten Dokumenten:
   "1-(2-Chlorphenyl)-5(4H)-tetrazolinon".

## Patentansprüche

1. Verfahren zur Herstellung von substituierten N-Carbamoyl-tetrazolinonen der Formel (I) 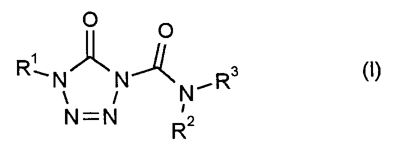 in welcher
R¹ für jeweils gegebenenfalls substituiertes Alkyl, Alkenyl, Alkinyl, Cycloalkyl, Cycloalkylalkyl, Aryl, Arylalkyl, Heterocyclyl oder Heterocyclylalkyl steht,
R² für jeweils gegebenenfalls substituiertes Alkyl, Alkenyl, Alkinyl oder Alkoxy steht und
R³ für jeweils gegebenenfalls substituiertes Alkyl, Alkenyl, Alkinyl, Cycloalkyl, Cycloalkylalkyl, Aryl oder Arylalkyl steht
oder zusammen mit R² für Alkandiyl steht,
dadurch gekennzeichnet, daß man
(a) Tetrazolinone der allgemeinen Formel (II) 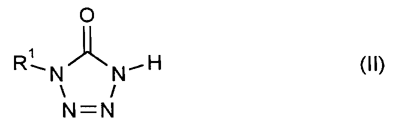 in welcher
R¹ die oben angegebene Bedeutung hat,
mit Carbamidsäurehalogeniden der allgemeinen Formel (III) 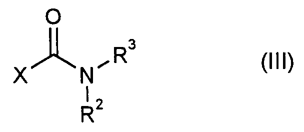 in welcher
R² und R³ die oben angegebene Bedeutung haben und
X für Halogen steht,
in Gegenwart eines Säureakzeptors und in Gegenwart eines Verdünnungsmittels bei Temperaturen zwischen 0°C und 200°C umsetzt und das hierbei als Nebenprodukt gebildete O-Carbamoylierungsprodukt der Formel (Ia) - unten - entweder durch Erhitzen zum gewünschten Produkt der Formel (I) isomerisiert oder durch Hydrolyse in wasserlösliche und somit leicht abtrennbare Komponenten überführt,
oder daß man
(b) (isolierte) Tetrazolinon-Metallsalze der allgemeinen Formel (IIa) 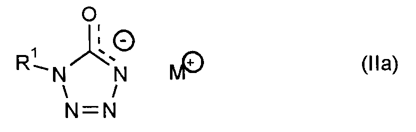 in welcher
R¹ die oben angegebene Bedeutung hat und
M für ein Alkalimetall-, ein Erdalkalimetall- oder ein Erdmetall-äquivalent steht,
mit Carbamidsäurehalogeniden der allgemeinen Formel (III) 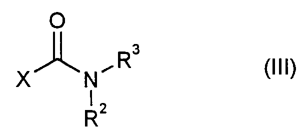 in welcher
R² und R³ die oben angegebene Bedeutung haben und
X für Halogen steht,
in Gegenwart eines Verdünnungsmittels bei Temperaturen zwischen 0°C und 200°C umsetzt und das hierbei als Nebenprodukt angefallene O-Carbamoylierungsprodukt der Formel (Ia) - unten - entweder durch Erhitzen zum gewünschten Produkt der Formel (I) isomerisiert oder durch Hydrolyse in wasserlösliche und somit leicht abtrennbare Komponenten überführt,
oder daß man
(c) substituierte Carbamoyloxytetrazole der allgemeinen Formel (Ia) 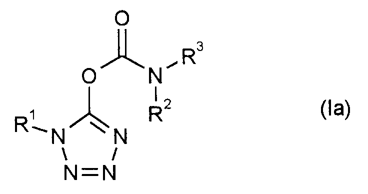 in welcher
R¹, R² und R³ die oben angegebenen Bedeutungen haben,
- welche auch in Mischung mit den Verbindungen der Formel (I) vorliegen können -
gegebenenfalls in Gegenwart eines Verdünnungsmittels und gegebenenfalls in Gegenwart eines Reaktionshilfsmittels bei Temperaturen zwischen 50°C und 200°C zu den entsprechenden N-Carbamoyl-tetrazolinonen der Formel (I) isomerisiert,
oder daß man
(d) substituierte Carbamoyloxytetrazole der allgemeinen Formel (Ia) 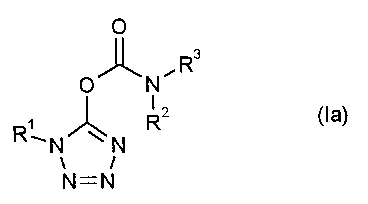 in welcher
R¹, R² und R³ die oben angegebenen Bedeutungen haben,
- welche als Produkte der Umsetzung von Verbindungen der Formel (II) bzw. von Verbindungen der Formel (IIa) mit Verbindungen der Formel (III) in Mischung mit den Verbindungen der Formel (I) vorliegen -
mit Wasser und/oder einem Alkohol, insbesondere Methanol, gegebenenfalls in Gegenwart eines inerten organischen Lösungsmittels und gegebenenfalls in Gegenwart eines Reaktionshilfsmittels bei Temperaturen zwischen 10°C und 150°C umsetzt und die Produkte der Hydrolyse bzw. Alkoholyse der Verbindungen der Formel (Ia) nach üblichen Methoden abtrennt.

2. Verfahren nach Anspruch 1, dadurch gekennzeichnet, daß in den Formeln (I), (Ia), (II), (IIa) und (III) jeweils
R¹ für gegebenenfalls durch Carboxy, Cyano, Carbamoyl, Halogen, C₁-C₆-Alkoxy, C₁-C₆-Alkylthio, C₁-C₆-Alkylsulfinyl oder C₁-C₆-Alkylsulfonyl substituiertes Alkyl mit 1 bis 10 Kohlenstoffatomen, für jeweils gegebenenfalls durch Carboxy, Cyano, Carbamoyl oder Halogen substituiertes Alkenyl oder Alkinyl mit jeweils 2 bis 10 Kohlenstoffatomen, für jeweils gegebenenfalls durch Carboxy, Cyano, Carbamoyl, Halogen, C₁-C₄-Alkyl oder C₁-C₄-Alkoxy-carbonyl substituiertes Cycloalkyl oder Cycloalkylalkyl mit jeweils 3 bis 8 Kohlenstoffatomen im Cycloalkylteil und gegebenenfalls 1 bis 4 Kohlenstoffatomen im Alkylteil, für jeweils gegebenenfalls durch Carboxy, Cyano, Carbamoyl, Nitro, Amino, Hydroxy, Halogen, durch C₁-C₄-Alkyl, C₁-C₄-Alkoxy, C₁-C₄-Alkylthio, C₁-C₄-Alkylsulfinyl, C₁-C₄-Alkylsulfonyl, Di-(C₁-C₄-alkyl)amino, C₁-C₄-Alkylsulfonylamino, Di-(C₁-C₄-alkyl)aminosulfonyl, C₁-C₄-Alkyl-carbonyl, C₁-C₄-Alkyl-carbonylamino, C₁-C₄-Alkoxy-carbonyl, Di-(C₁-C₄-alkylamino)-carbonyl, C₁-C₄-Alkylendioxy, Phenyl oder Phenoxy (welche jeweils gegebenenfalls durch Fluor und/oder Chlor substituiert sind) substituiertes Phenyl, Naphthyl, Phenyl-C₁-C₄-alkyl, Naphthyl-C₁-C₄-alkyl, Furyl, Benzofuryl, Tetrahydrofuryl, Furylmethyl, Tetrahydrofurylmethyl, Thienyl, Benzothienyl, Tetrahydrothienyl, Thienylmethyl, Tetrahydrothienylmethyl, Pyrrolyl, Indolyl, Oxazolyl, Benzoxazolyl, Oxazolylmethyl, Isoxazolyl, Isoxazolylmethyl, Thiazolyl, Benzthiazolyl, Thiazolylmethyl, Pyrazolyl, Oxadiazolyl, Oxadiazolylmethyl, Thiadiazolyl, Thiadiazolylmethyl, Triazolyl, Pyridyl, Chinolinyl, Isochinolinyl, Pyridylmethyl, Pyrazinyl, Pyridazinyl, Pyrimidinyl, Chinazolinyl, Chinoxalinyl, Pyrimidinylmethyl, Triazinyl oder Triazinylmethyl steht,
R² für jeweils gegebenenfalls durch Cyano oder Halogen substituiertes Alkyl, Alkenyl oder Alkinyl mit jeweils bis zu 6 Kohlenstoffatomen steht und
R³ für jeweils gegebenenfalls durch Cyano oder Halogen substituiertes Alkyl mit 1 bis 6 Kohlenstoffatomen, für für jeweils gegebenenfalls durch Cyano oder Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 2 Kohlenstoffatomen im Alkylteil, oder für jeweils gegebenenfalls durch Cyano, Nitro, Halogen, durch C₁-C₄-Alkyl, C₁-C₄-Alkoxy, C₁-C₄-Alkylthio, C₁-C₄-Alkylsulfinyl, C₁-C₄-Alkylsulfonyl, Di-(C₁-C₄-alkyl)amino, C₁-C₄-Alkylsulfonylamino, Di-(C₁-C₄-alkyl)aminosulfonyl, C₁-C₄-Alkyl-carbonyl oder C₁-C₄-Alkoxy-carbonyl (welche jeweils gegebenenfalls durch Fluor und/oder Chlor substituiert sind) substituiertes Phenyl oder Phenyl-C₁-C₂-alkyl steht,
oder zusammen mit R² für Alkandiyl mit 2 bis 6 Kohlenstoffatomen steht,
daß in Formel (IIa)
M für ein Lithium-, Natrium-, Kalium-, Magnesium-, Calcium-, Barium- oder Aluminium-äquivalent steht,
und daß in Formel (III)
X für Fluor, Chlor oder Brom steht.

3. Verfahren zur Herstellung von 1-(2-Chlorphenyl)-4-(N-cyclohexyl-N-ethyl-aminocarbonyl)-1,4-dihydro-5H-tetrazol-5-on gemäß Anspruch 1.

4. Tetrazolinon-Metallsalze der Formel (IIa) 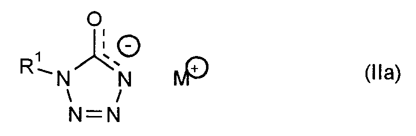 in welcher
R¹ für jeweils gegebenenfalls substituiertes Alkyl, Alkenyl, Alkinyl, Cycloalkyl, Cycloalkylalkyl, Aryl, Arylalkyl, Heterocyclyl oder Heterocyclylalkyl steht,
R² für jeweils gegebenenfalls substituiertes Alkyl, Alkenyl, Alkinyl oder Alkoxy steht und
R³ für jeweils gegebenenfalls substituiertes Alkyl, Alkenyl, Alkinyl, Cycloalkyl, Cycloalkylalkyl, Aryl oder Arylalkyl steht
oder zusammen mit R² für Alkandiyl steht, und
M für ein Alkalimetall-, ein Erdalkalimetall- oder ein Erdmetall-äquivalent steht.

5. Substituierte Carbamoyloxytetrazole der Formel (Ia) 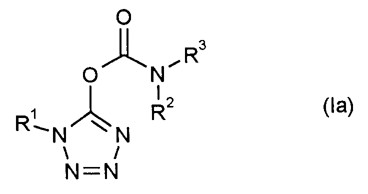 in welcher
R¹ für jeweils gegebenenfalls substituiertes Alkyl, Alkenyl, Alkinyl, Cycloalkyl, Cycloalkylalkyl, Aryl, Arylalkyl, Heterocyclyl oder Heterocyclylalkyl steht,
R² für jeweils gegebenenfalls substituiertes Alkyl, Alkenyl, Alkinyl oder Alkoxy steht und
R³ für jeweils gegebenenfalls substituiertes Alkyl, Alkenyl, Alkinyl, Cycloalkyl, Cycloalkylalkyl, Aryl oder Arylalkyl steht
oder zusammen mit R² für Alkandiyl steht.
